# EUROPÄISCHE PATENTSCHRIFT

(11) **EP 1 225 850 B1**
(45) Veröffentlichungstag und Bekanntmachung des Hinweises auf die Patenterteilung: **11.06.2008**
(21) Anmeldenummer: 00977441.5
(22) Anmeldetag: 30.10.2000
(51) Int. Cl.: A61F 2/44, A61F 2/46, A61L 27/36

(54) **IMPLANTAT AUS KNOCHENMATERIAL**
IMPLANT CONSISTING OF BONE MATERIAL
IMPLANT EN MATIERE OSSEUSE

(30) Priorität: 03.11.1999 DE 19952939
(43) Veröffentlichungstag der Anmeldung: 31.07.2002
(73) Patentinhaber: Tutogen Medical GmbH, 91077 Neunkirchen am Brand (DE)
(72) Erfinder: SCHÖPF, Christoph, 91096 Möhrendorf (DE); KOSCHATZKY, Karl, 91058 Erlangen (DE); KALAS, Rolf-Dieter, 34320 Söhrewald 1 (DE); LÖWEL, Matthias, 90408 Nürnberg (DE)
(74) Vertreter: Manitz, Finsterwald & Partner GbR
(86) Internationale Anmeldenummer: PCT/EP2000/010672
(87) Internationale Veröffentlichungsnummer: WO 2001/032110

(56) Entgegenhaltungen:
- WO-A-00/40179
- WO-A-99/09914
- DE-A- 2 906 650
- US-A- 5 514 180
- US-A- 5 861 041

## Beschreibung

Die vorliegende Erfindung betrifft ein Implantat zur Verbindung von Knochen und insbesondere ein Wirbelsäulenimplantat zur interkorporellen Fusion von Wirbelknochen, das zwischen zwei zu fusionierende Wirbelknochen eingesetzt wird.

Durch die Degeneration der Bandscheibe, insbesondere des Bandscheibenkerns (Nucleus pulposus) kommt es zu einem Höhenverlust im betroffenen Bandscheibenfach, der mit einer Lockerung des Bandscheibenringes (Annulus fibrosus) und der Bänder verbunden ist. Dadurch wird die Wirbelsäule an dieser Stelle instabil. Die Folge ist eine horizontale Verschiebbarkeit der Wirbelkörper gegeneinander (Spondylolisthese), die zu Beeinträchtigungen der Nervenwurzeln in diesem Bereich und oder des Rükkenmarks mit daraus resultierenden Schmerzen führt. Ähnliche Symptome können nach chemisch-enzymatischer oder physikalischer (Laser) Auflösung des Bandscheibenkerns (Nucleolyse) zur Behandlung eines Bandscheibenvorfalls auftreten (Postnukleolyse-Syndrom).

Das Prinzip zur Behandlung dieser Symptome besteht in der operativen Ausräumung des Bandscheibenkerns und dem Einlegen bzw. Einschieben - im Bereich der Halswirbelsäule - eines oder - im Bereich der Lendenwirbelsäule - zweier ausreichend stabiler Körper, um die normale Höhe des Bandscheibenfaches wiederherzustellen. Gleichzeitig muß die horizontale Verschiebbarkeit verhindert werden. Dies geschieht entweder durch das Implantat selbst oder durch zusätzliche Metallimplantate (instrumentierte Fusion). Diese Implantate unterliegen insbesondere in der Lendenwirbelsäule erheblichen Kräften, die zum Bruch des Metallimplantats führen können. Es wird daher angestrebt, daß das Zwischenwirbelinterponat möglichst rasch und solide mit den angrenzenden Wirbelkörpern verwächst bzw. fusioniert.

Zur Behandlung von Patienten mit spinalem Trauma oder degenerativen Leiden an der Wirbelsäule werden im wesentlichen zwei Techniken angewendet.
1. Entfernen des Bandscheibenkerns und des Knorpels an den Endplatten, Aufdehnen des Zwischenwirbelraumes auf normale Weite und Einschieben eines planparallelen oder horizontal leicht keilförmigen Blocks (Smith-Robinson-Technik).
2. Aufdehnen des Bandscheibenfaches auf normale Höhe, Bohren eines zylindrischen Loches, das beide Wirbelkörper erfaßt, und Einschieben eines zylindrischen Dübels (Cloward-Technik). Der Dübel kann dabei entweder ein glatter Zylinder sein oder ein Gewinde aufweisen.

Ein relativ neues Verfahren zur interkorporellen Fusion an der Lendenwirbelsäule ist die posteriore lumbale interkorporelle Fusion in unilateraler transforaminaler Technik. Hierbei wird das Foramen intervertebrale des betroffenen Segmentes einseitig eröffnet, das Bandscheibenfach wird ausgeräumt und es werden zur ventralen Abstützung zwei auf passende Höhe zugeschnittene Titangitterkörbe eingebracht, welche mit Knochenchips gefüllt sind. Diese gewebeschonende Methode hat jedoch verschiedene Nachteile. Zum einen können die scharfen Kanten der Titankörbe beim Einbringen die Nervenwurzeln beschädigen. Ferner ist eine Lordosierung nicht möglich, da die Körbe praktisch blind eingebracht werden. Schließlich stehen nur die Ränder der Körbe zur Verfügung, um den vorhandenen Druck aufzunehmen. Das Titan erschwert durch Artefakte und in der Computertomographie die Fusionskontrolle. Darüberhinaus ist die Implantatentfernung bei Revisionsoperationen außerordentlich schwierig.

Aus der US 5,861,041 ist ein zweiteiliges Wirbelsäulenimplantat bekannt, das im verbundenen Zustand eine im wesentlichen elliptische Konfiguration besitzt.

Die WO 99/09914 beschreibt ein Wirbelsäulenimplanat nach dem Oberbegriff des Anspruchs 1.

Die Aufgabe der vorliegenden Erfindung besteht deshalb darin, ein Implantat zur Fusion von Knochen zu schaffen, das die oben genannten Nachteile beseitigt.

Die Lösung dieser Aufgabe erfolgt durch die Merkmale des Anspruchs 1.

Ein besonderer Vorteil des Wirbelsäulenimplantats gemäß der Erfindung ist durch das verwendete Material gegeben, das aufgrund seines biologischen Ursprungs und einer speziellen Konservierung keinen Fremdkörper darstellt. Darüberhinaus ergibt sich durch die gekrümmte Gestaltung des bananenförmige Krümmung auf. In einer besonders vorteilhaften Ausgestaltung weist der Körper abgerundete Kanten auf. Eine solche Gestaltung des Körpers erleichtert die Applikation des Wirbelsäulenimplantats zwischen den zu fusionierenden Wirbelkörpern, indem die Abrundungen ein Verkanten des Implantats während der Applikation vermeiden. Durch die spezielle Bananenform ist das Implantat besonders gut an die natürliche Form der Wirbelkörperendplatten angepaßt und bietet so die größtmögliche Kontaktfläche zu den Endplatten. Dadurch erfolgt eine mehr physiologische Verteilung der Kräfte wodurch Druckspitzen und ein durch diese bedingtes Einsinken des Implantates in die Wirbelkörper vermieden werden. Da durch die Krümmung des Implantates automatisch eine korrekte Positionierung erfolgt, ist durch einen trapezförmigen Querschnitt auch die Wiederherstellung der physiologischen Krümmung der Wirbelsäule - Lordose - möglich.

Besonders vorteilhaft ist es, daß die Einkerbungen oder Vertiefungen die Mitnahme von lose eingebrachtem Knochenpulver in den Zwischenwirbelraum ermöglichen. Auf diese Weise kann beim Einschieben des Implantats in den Zwischenwirbelraum automatisch Knochenpulver in den konkav gekrümmten Bereich des Implantats eingebracht werden.

Nach einem weiteren Aspekt der vorliegenden Erfindung sind Aufnahmeöffnungen auf einer oder mehreren Seiten des Körpers als Bohrloch mit einem Gewinde von vorzugsweise 3 mm Tiefe ausgeführt. In diese Gewindebohrungen können dann mit passendem Gewinde versehene Applikationswerkzeuge eingeschraubt werden, um das Implantat positionsgenau zwischen die zu fusionierenden Wirbel einzubringen.

Als Material wird für das Wirbelsäulenimplantat gemäß der vorliegenden Erfindung ein geeignetes allogenes oder xenogenes Knochenmaterial derart prozessiert, daß es konserviert, lagerfähig sowie steril ist und bestimmungsgemäß eingesetzt werden kann. Die Konservierung des Knochenmaterials kann beispielsweise mittels Gefriertrocknung erfolgen. Ein anderes bevorzugtes Verfahren der Herstellung des Knochenmaterials ist die Prozessierung durch vorzugsweise Lösungsmitteldehydratisierung von nativem Knochenmaterial mittels eines organischen, mit Wasser mischbaren Lösungsmittels, z.B. Methanol, Äthanol, Propanol, Isopropanol, Aceton, Methyl-Äthylketon oder Gemischen dieser Lösungsmittel. Die Konservierung und Sterilisation des Knochenmaterials nach diesem Verfahren ist auch Gegenstand des Patents DE 29 06 650.

Dieses Verfahren dient der Herstellung von Transplantatkonserven und ermöglicht eine Dehydratisierung und Freilegung bis in den Feinbau der Fibrille des Knochenmaterials, so daß das prozessierte Knochenmaterial im histologischen Bild eine dem natürlichen Knochen sehr ähnliche morphologische Struktur aufweist und somit die gewünschten Eigenschaften des Knochenmaterials erhalten bleiben. Dieses Verfahren der Lösungsmitteldehydratisierung hat außerdem den Vorteil, daß im Vergleich zur Gefriertrocknung ein wesentlich geringerer apparativer Aufwand erforderlich ist.

Ferner kann das Knochenmaterial auch durch Lösungsmitteldehydratisierung von nativem Knochen mit anschließender terminaler Sterilisation, insbesondere durch Bestrahlung mit Gamma-Strahlen erzeugt werden. Alternativ kann das spongiöse Knochenmaterial durch aseptische Prozessierung von Knochenmaterial ohne terminale Sterilisation erzeugt werden. Das Ausgangsmaterial des erfindungsgemäßen Knochenimplantats ist menschlicher oder tierischer Knochen von ausreichender Größe.

Zur Entfernung der Antigenität wird der Knochen einer osmotischen Behandlung unterzogen. Weiterhin wird zur Denaturierung löslicher Proteine eine oxidative Behandlung durchgeführt. Zur Optimierung der Virusinaktivierung kann eine pH-Absenkung auf pH 3 erfolgen oder eine Behandlung mit Natronlauge oder einer anderen DNA/RNA zerstörenden Substanz. Der Wasserentzug erfolgt durch organische Lösungsmittel, vorzugsweise Azeton. Die abschließende Sterilisation erfolgt durch energiereiche Strahlung, vorzugsweise γ-Strahlen mit einem Dosismaximum von 25 kGy.

Ein so behandelter Knochen behält seinen natürlichen Mineral-Collagen-Verbund und Eigenschaften. Darüberhinaus ist ein so behandelter Knochen umbaufähig.

Nachfolgend wird die vorliegende Erfindung rein exemplarisch anhand von Ausführungsbeispielen eines erfindungsgemäßen Wirbelsäulenimplantats unter Bezugnahme auf die beigefügten Zeichnungen beschrieben. Es zeigen:
- Fig. 1: eine Draufsicht auf eine Ausführungsform eines Wirbelsäulenimplantats gemäß der vorliegenden Erfindung;
- Fig. 2: eine bekannte Querschnittsform eines Wirbelsäulenimplantats;
- Fig. 3: eine mögliche Querschnittsform der erfindungsgemäßen Wirbelsäulenimplantate;
- Fig. 4: eine Draufsicht auf eine weitere Ausführungsform eines Wirbelsäulenimplantats gemäß der vorliegenden Erfindung; und
- Fig. 5: eine Draufsicht auf eine weitere Ausführungsform eines Wirbelsäulenimplantats gemäß der vorliegenden Erfindung.

In den Figuren bezeichnen die gleichen Bezugszeichen jeweils die gleichen Komponenten der dargestellten Ausführungsformen.

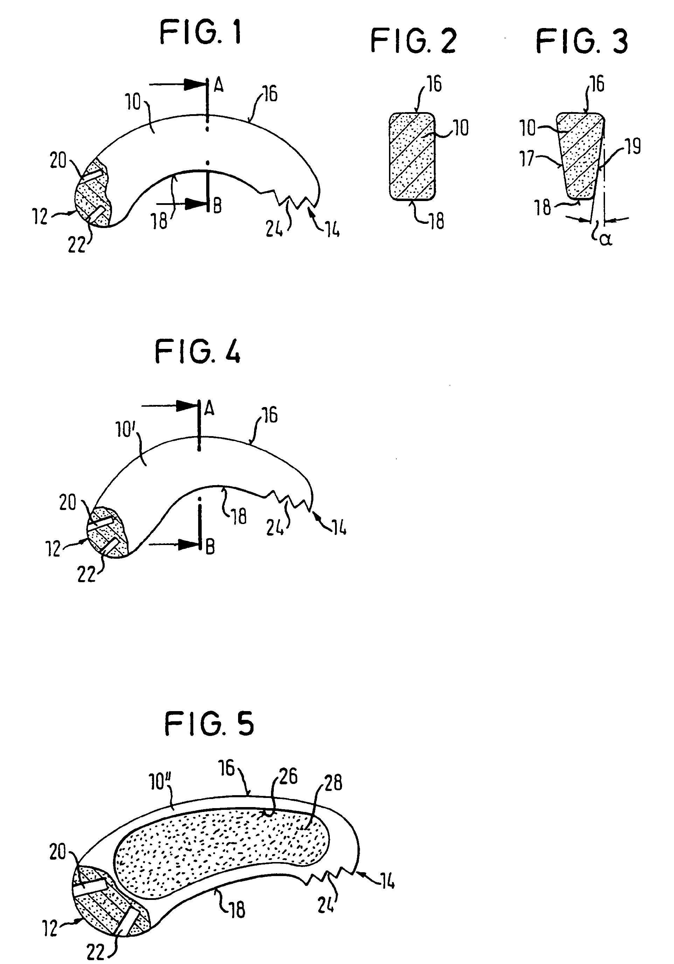

Das in Fig. 1 dargestellte Ausführungsbeispiel eines erfindungsgemäßen Wirbelsäulenimplantats umfaßt einen Körper 10, der beispielsweise aus kortikalem, diaphysärem Knochenmaterial z.B. humanen Ursprungs besteht. Dieser Körper 10 ist in Draufsicht grundsätzlich langgestreckt und in Richtung seiner Längserstreckung gekrümmt ausgebildet.

Die beiden entgegengesetzten Enden 12 und 14 des Körpers 10 sind abgerundet ausgebildet, so daß sich insgesamt eine bananenförmige Gestaltung des Grundkörpers 10 ergibt. Zwischen den beiden Enden 12 und 14 besitzt der Körper 10 eine konvex gekrümmte Fläche 16 und eine konkav gekrümmte Fläche 18.

Wie Fig. 1 zeigt, weist der Grundkörper im Bereich seines einen Endes 12 zwei Aufnahmeöffnungen 20, 22 für Applikationswerkzeuge auf. Die Aufnahmeöffnungen 20, 22 sind als Bohrungen ausgebildet, deren Längsachsen nicht zueinander parallel verlaufen.

Im Bereich des gegenüberliegenden Endes 14 ist eine Strukturierung der Oberfläche des Grundkörpers im Bereich der konkaven Fläche 18, vorzugsweise durch Einkerbungen bzw. Vertiefungen 24 vorgesehen, welche die Mitnahme von lose zugegebenem Knochenpulver in den Zwischenwirbelraum ermöglichen. Durch die vorgesehene Oberflächenstrukturierung (in dem dargestellten Ausführungsbeispiel sind drei Einkerbungen vorgesehen) ist die Mitnahme und Verdichtung des Knochenpulvers beim Einbringen des Knochemimplantates in den Zwischenwirbelraum gewährleistet.

Die Fig. 2 und 3 zeigen mögliche Querschnittsformen des Körpers 10 entlang der Schnittlinie A-B von Fig. 1.

Fig. 2 zeigt eine mögliche Querschnittsform in Form eines Rechteckes mit abgerundeten Kanten. Fig. 3 zeigt eine trapezförmige Querschnittsform, wobei die schräg verlaufenden Seiten des Trapezes unter einem Winkel α von etwa 3° bis 6° von der Rechteckform abweichen.

Wie insbesondere Fig. 3 zeigt, können bei trapezförmigem Querschnitt des Implantats die schräg verlaufenden Außenseiten 17, 19 in Richtung der konkaven Außenfläche 18 geneigt sein, d. h. in Richtung der Ringmitte des ringsegmentförmig geformten Implantats.

Fig. 1 zeigt eine im wesentlichen symmetrische Ausführungsform eines Wirbelsäulenimplantats. Demgegenüber ist bei der in Fig. 4 dargestellten weiteren Ausführungsform eine asymmetrische Formgebung gewählt, bei welcher die Form des Körpers 10' sich in Draufsicht von dem einen Ende 12 in Richtung des anderen Endes 14 verjüngt. Im übrigen bezeichnen gleiche Bezugszeichen gleiche Elemente. Gleichzeitig können die in den Fig. 2 und 3 dargestellten Querschnittsformen auch bei der in Fig. 4 dargestellten Ausführungsform Anwendung finden.

Wie die Fig. 1 und 4 zeigen, besitzen die Körper 10, 10' die Form eines Ringsegmentes, wobei bevorzugt die Form eines etwa 3/8 bis 1/2 Ringes gewählt wird. Ferner ist bei der Ausführungsform von Fig. 4 zu erkennen, daß das eine Ende 12 des Körpers 10' breiter ist als das gegenüberliegende Ende 14, um ein Einführen des Implantats in das Foramen intervertebrale zu erleichtern.

Grundsätzlich ist der Körper 1 des Wirbelsäulenimplantats in seiner Größe an den vorgegebenen Platz, an dem das Implantat eingesetzt werden soll, angepaßt. Die Außenabmessungen eines solchen Wirbelsäulenimplantats können bei lumbaler Anwendung beispielsweise folgendermaßen sein: Länge etwa 30 bis 60 mm, Breite etwa 8 bis 20 mm, Höhe etwa 6 bis 18 mm.

Grundsätzlich besteht die Möglichkeit, daß das erfindungsgemäße Wirbelsäulenimplantat massiv ausgebildet wird, beispielsweise aus kortikalem oder aus spongiösem Knochen. Eine alternative Ausführungsform der Erfindung sieht vor, daß das Wirbelsäulenimplantat - ebenfalls aus kortikalem oder spongiösem Knochen - als Hohlkörper ausgebildet wird.

Fig. 5 zeigt eine solche Ausführungsform, bei welcher der Körper 10" einen Hohlraum 26 aufweist, der mit spongiösem Knochen 28 gefüllt ist. Der Hohlraum 26 erstreckt sich im wesentlichen vom Ende der Aufnahmeöffnungen 20, 22 bis in den Bereich der Vertiefungen 24. Hierbei ist der Hohlraum 26 an die Außenkontur des Körpers 10" angepaßt.

### Bezugszeichenliste

- 10, 10', 10": Körper
- 12, 14: Ende
- 16: konvexe Außenseite
- 17: Außenseite
- 18: konkave Außenseite
- 19: Außenseite
- 20, 22: Aufnahmeöffnung
- 24: Vertiefungen
- 26: Hohlraum
- 28: spongiöser Knochen
- α: Neigungswinkel

## Patentansprüche

1. Wirbelsäulenimplantat zur interkorporellen Fusion an der Wirbelsäule, bestehend aus einem Körper (10, 10', 10") aus Knochenmaterial, der einen rechteckigen oder trapezförmigen Querschnitt besitzt, und der in Richtung seiner Längserstreckung gekrümmt ausgebildet ist, wodurch eine konvex gekrümmte Seite (16) und eine konkav gekrümmte Seite (18) gebildet ist, und wobei das Implantat in seiner Größe an den nach Ausräumung des Wirbelzwischenkörpers vorhandenen Wirbelzwischenraum zwischen benachbarten Wirbeln angepaßt ist, **dadurch gekennzeichnet,**
**dass** an einem Ende des Körpers (10, 10', 10") Einkerbungen oder Vertiefungen (24) im Bereich der konkav gekrümmten Seite (18) vorgesehen sind, welche die Mitnahme von lose eingebrachtem Knochenpulver in den Wirbelzwischenraum ermöglichen.

2. Wirbelsäulenimplantat nach Anspruch 1
**dadurch gekennzeichnet,**
**daß** der Körper (10, 10', 10") aus konserviertem und sterilem bovinem Knochenmaterial humanen oder tierischen Ursprungs besteht.

3. Wirbelsäulenimplantat nach einem der vorangehenden Ansprüche
**dadurch gekennzeichnet,**
**daß** zumindest ein Ende (12, 14) des Körpers (10, 10', 10") in Draufsicht halbkreisförmig abgerundet ist.

4. Wirbelsäulenimplantat nach einem der vorangehenden Ansprüche
**dadurch gekennzeichnet,**
**daß** dieses mindestens eine Aufnahmeöffnung (20, 22) für ein Applikationswerkzeug aufweist.

5. Wirbelsäulenimplantat nach einem der vorangehenden Ansprüche
**dadurch gekennzeichnet,**
**daß** der Körper (10, 10', 10") abgerundete Kanten aufweist.

6. Wirbelsäulenimplantat nach einem der vorangehenden Ansprüche
**dadurch gekennzeichnet,**
**daß** der Körper (10, 10', 10") die Form eines Ringsegmentes besitzt, welches etwa 3/8 bis 1/2 eines Ringes ist.

7. Wirbelsäulenimplantat nach einem der vorangehenden Ansprüche,
**dadurch gekennzeichnet,**
**daß** ein Ende (14) des Körpers (10') schmaler ist als das andere Ende (12).

## Claims

1. A spinal column implant for intercorporal fusion at the spinal column consisting of a body (10, 10', 10") of bone material, which has a rectangular or trapezoidal cross-section and which is made curved in the direction of its longitudinal extent, whereby one convexly curved side (16) and one concavely curved side (18) is formed, and wherein the implant is matched in its size to the intervertebral space present between adjacent vertebrae after removal of the intervertebral body, **characterized**
**in that** notches or recesses (24) are provided in the region of the concavely curved side (18) at one end of the body (10, 10',10") which allow the entraining of loosely introduced bone powder into the intervertebral space.

2. A spinal column implant in accordance with claim 1, **characterized in that** the body (10, 10', 10") consists of preserved and sterile bone material of human or animal origin.

3. A spinal column implant in accordance with any one of the preceding claims, **characterized in that** at least one end (12, 14) of the body (10, 10', 10") is rounded in plan view in semi-circular shape.

4. A spinal column implant in accordance with any one of the preceding claims, **characterized in that** it has at least one reception opening (20, 22) for an application tool.

5. A spinal column implant in accordance with any one of the preceding claims, **characterized in that** the body (10, 10',10") has rounded edges.

6. A spinal column implant in accordance with any one of the preceding claims, **characterized in that** the body has the form of a ring segment, preferably of approximately 3/8 to 1/2 of a ring.

7. A spinal column implant in accordance with any one of the preceding claims, **characterized in that** one end (14) of the body (10') is narrower than the other end (12).

## Revendications

1. Implant vertébral pour la fusion intercorporelle au niveau de la colonne vertébrale, comprenant un corps (10, 10', 10") en matériau osseux qui possède une section rectangulaire ou trapézoïdale et qui est réalisé de façon incurvée dans la direction de son extension longitudinale, grâce à quoi il se forme un côté à courbure convexe (16) et un côté à courbure concave (18), et l'implant est adapté quant à sa taille à l'intervalle intervertébral présent entre des vertèbres voisines après enlèvement du corps intervertébral,
**caractérisé en ce qu'**à une extrémité du corps (10, 10', 10") sont ménagées des entailles ou des renfoncements (24) dans la zone du côté à courbure concave (18), qui permettent d'entraîner jusque dans l'espace intervertébral de la poudre osseuse introduite à l'état lâche.

2. Implant vertébral selon la revendication 1,
**caractérisé en ce que** le corps (10, 10', 10") est réalisé en matériau osseux bovin conservé et stérile d'origine humaine ou animale.

3. Implant vertébral selon l'une des revendications précédentes,
**caractérisé en ce qu'**au moins une extrémité (12, 14) du corps (10, 10', 10") est arrondie en forme de demi-cercle en vue de dessus.

4. Implant vertébral selon l'une des revendications précédentes,
**caractérisé en ce que** celui-ci présente au moins une ouverture de réception (20, 22) pour un outil d'application.

5. Implant vertébral selon l'une des revendications précédentes,
**caractérisé en ce que** le corps (10, 10', 10") présente des arêtes arrondies.

6. Implant vertébral selon l'une des revendications précédentes,
**caractérisé en ce que** le corps (10, 10', 10") possède la forme d'un segment annulaire qui constitue approximativement 3/8 à 1/2 d'un anneau.

7. Implant vertébral selon l'une des revendications précédentes,
**caractérisé en ce qu'**une extrémité (14) du corps (10') est plus étroite que l'autre extrémité (12).
